# EUROPEAN PATENT SPECIFICATION

(11) **EP 2 512 424 B1**
(45) Date of publication and mention of the grant of the patent: **11.12.2019**
(21) Application number: 09801296.6
(22) Date of filing: 18.12.2009
(51) Int. Cl.: A61K 8/44, A61K 8/49, A61Q 5/12

(54) **PROCESS FOR TREATING KERATIN FIBERS**
BEHANDLUNGSVERFAHREN FÜR KERATINISCHE FASERN
PRODÉDÉ DE TRAITEMENT DE FIBRES KÉRATINIQUES

(43) Date of publication of application: 24.10.2012
(73) Proprietor: L'Oréal, 75008 Paris (FR)
(72) Inventor: DE BONI, Maxime, Kawasaki-shi Kanagawa 213-0012 (JP); TAKAHASHI, Hiroshi, Kawasaki-shi Kanagawa 213-0012 (JP)
(74) Representative: Lavoix
(86) International application number: PCT/JP2009/071726
(87) International publication number: WO 2011/074136

(56) References cited:
- EP-A1- 1 369 103
- EP-A2- 2 111 852
- DE-A1- 19 617 515
- JP-A- 2007 039 410
- US-A1- 2009 283 106
- KOYAMA T ET AL: "Hair cosmetics for use as hair conditioner, contains amino acid such as glycine, alanine, serine, proline, leucine and/or isoleucine, and polymerized amino modified silicones" WPI/THOMSON,, vol. 2006, no. 10, 26 January 2006 (2006-01-26), XP002536266

## Description

### TECHNICAL FIELD

The present invention relates to a process for treating keratin fibers such as hair, as well as a composition and a kit to be used for the process.

### BACKGROUND ART

Due to the many physical stresses (UV, shampoo, brushing) and chemical stresses (coloration, perm, relaxing, pollution) that keratin fibers such as hair must undergo daily, research for effectively repairing damaged keratin fibers has become important in the cosmetic treatments for keratin fibers.

Repairing damaged keratin fibers is only worthwhile if a real sensation of return to the original state of the keratin fibers is perceived. Furthermore, the repairing treatment should be effective against various stresses, as mentioned before.

Technologies (compositions and/or processes) known as treatments for repairing damaged keratin fibers that have been proposed are still insufficient insofar as they are often temporary and do not achieve proper recovery of keratin fiber integrity.

Amino acids which are components of proteins included in the keratin fibers are commonly used in hair care products. For example, please refer to US-A-2006-263315.

### DISCLOSURE OF INVENTION

The main expectations of using amino acids are generally the reconstruction of damaged keratin fibers, and the recovery of cosmetic properties for damaged keratin fiber surface.

Nevertheless, their benefits on keratin fibers are still insufficient due to their low deposition yield onto the keratin fibers and low penetration rate into the keratin fibers. This means that the efficiency of the cosmetic treatment of keratin fibers using an amino acid is low and a large amount of amino acid is necessary in the formulation used for the cosmetic treatment.

Thus, an objective of the present invention is to provide a new treatment process for keratin fibers such as hair, using an amino acid except for cystein, basic arginine and basic lysine, even with a relatively small amount thereof, which provides the keratin fibers with good cosmetic effects, in particular superior repairing or recovering effects, which can be effective against various stresses for a long time.

The above objective of the present invention can be achieved by a process as disclosed in claim 1.

It is also described a process for treating keratin fibers comprising the steps of:
applying onto the keratin fibers a composition comprising at least one amino acid except for cystein, basic arginine and basic lysine;
   then placing the keratin fibers in an occlusive space; and then heating the keratin fibers,
wherein
the composition contains neither a reducing agent nor a source of carbonate ions of the formula: 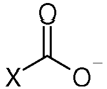 wherein
   X is a group selected from the group consisting of O⁻, OH, NH₂, O-OH, and O-COO⁻.

The above process may further comprise the step of rinsing the keratin fibers after the step of applying the composition onto the keratin fibers and/or after the step of heating the keratin fibers.

Mechanical tension is provided to the keratin fibers. The mechanical tension may be provided by using at least one reshaping means selected from the group consisting of a curler, a roller, a plate and an iron.

The occlusive space is formed by at least one coating means.

The coating means is flexible. The coating means comprises at least one member selected from the group consisting of a film and a sheet.

In the above process, the keratin fibers is heated at 60°C to 250ºC during the step of heating the keratin fibers. The keratin fibers may be heated by at least one heater providing at least one selected from the group consisting of hot air, hot steam, high frequency induction heating, microwave heating, infrared ray irradiation, laser, and flash lamp irradiation. The coating means and/or the reshaping means may comprise the heater.

The amino acid may be an alpha-amino acid.

The amino acid may correspond to the formula: 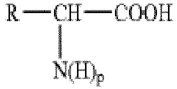 in which
when p=2, R represents a hydrogen atom, an aliphatic group optionally containing one or several nitrogen atoms, a heterocyclic portion, or an aromatic group, or when p=1, R can form a heterocycle with the nitrogen atom of -N(H)ₚ.

The amino acid may be selected from the group consisting of aspartic acid, glutamic acid, alanine, asparagine, glutamine, glycine, histidine, leucine, isoleucine, methionine, N-phenylalanine, proline, hydroxyproline, serine, threonine, tryptophan, tyrosine, valine, and mixtures thereof.

The composition comprises the amino acid in an amount of 0.005 to 20% by weight relative to the total weight of the composition. the pH of the composition may range from 6 to 11.

It is also described a composition for treating keratin fibers to be heated in an occlusive space, comprising at least one amino acid except for cystein, basic arginine and basic lysine,
wherein
the composition contains neither a reducing agent nor a source of carbonate ions of the formula: 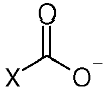 wherein
X is a group selected from the group consisting of O⁻, OH, NH₂, O-OH, and O-COO⁻.

It is also described a kit for treating keratin fibers, comprising:
a device comprising
at least one coating means to form an occlusive space, and
at least one heater to heat the keratin fibers in the occlusive space;
and
a composition comprising at least one amino acid except for cystein, basic arginine and basic lysine,
wherein
the composition contains neither a reducing agent nor a source of carbonate ions of the formula: 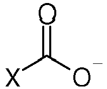 wherein
   X is a group selected from the group consisting of O⁻, OH, NH₂, O-OH, and O-COO⁻.

### BEST MODE FOR CARRYING OUT OF THE INVENTION

After diligent research, the inventors have discovered that it is possible to provide keratin fibers such as hair with good cosmetic effects, using an amino acid, and in particular superior repairing or recovering effects, which can be effective against various stresses for a long time, by using a composition comprising at least one amino acid in association with a specific heating process for the keratin fibers.

The above specific heating process is performed in a closed or occlusive environment, which limits the evaporation of water or moisture from the keratin fibers and maintains the keratin fibers at higher temperature preferably in the wet state. Accordingly, the keratin fibers can be evenly heated, and the amino acid can easily penetrates into or deposits onto the keratin fibers such that it can remain in or on the keratin fibers for a long time even after some stresses such as shampooing.

Since an amino acid can easily stay on or in the keratin fibers, the process according to the present invention can exhibit good cosmetic effects, in particular good mechanical properties, by using even a relatively small amount of amino acid as compared to a conventional process in which it is difficult for an amino acid to stay on or in the keratin fibers.

The composition used in the process of the present invention must not contain any reducing agents such as thiol-compounds. Therefore, malodor derived from the reducing agents can be prevented. Furthermore, the composition used in the process of the present invention must not contain any carbonate ion source as defined above. Therefore, cosmetic treatment is more effective, because there is no possibility of producing carbon dioxide which may form a foam that may inhibit the deposition or penetration of the amino acid on or into the keratin fibers.

### (Composition)

The composition used in the process of the present invention comprises at least one amino acid except for cystein, basic arginine and basic lysine.

The term "amino acid" here means a compound which is not obtained by polycondensation of identical or different amino acids. In addition, the term "amino acid" here encompasses not only an amino acid itself but also an amino acid in the form of a salt thereof. As the salt, mention may be made of sodium salt, magnesium salt, potassium salt, and calcium salt.

The amino acids that may be used according to the present invention comprise at least one amine function and at least one acid function.

The acid function(s) may be carboxylic, sulfonic, phosphonic or phosphoric, and are preferably carboxylic.

The amino acids that may be used according to the present invention may be α-amino acids, β-amino acids, or γ-amino acids. Preferably, the amino acids used in the present invention are α-amino acids, i.e., they comprise an amine function and an acid function at the same carbon atom.

The α-amino acids may be represented by the following formula: 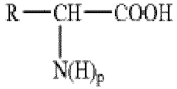 in which:
when p=2, R represents a hydrogen atom, an aliphatic group optionally containing one or several nitrogen atoms, a heterocyclic portion, or an aromatic group, or
when p=1, R can form a heterocycle with the nitrogen atom of - N(H)ₚ. This heterocycle is preferably a saturated 5-membered ring, optionally substituted with one or more C₁₋₄ alkyl or hydroxyl groups.

Preferably, the aliphatic group is a linear or branched C₁₋₄ alkyl group; a linear or branched C₁₋₄ hydroxyalkyl group; a linear or branched C₁₋₄ aminoalkyl group; a linear or branched (C₁₋₄ alkyl) thio(C₁₋₄) alkyl group; a linear or branched C₂₋₄ carboxyalkyl group; a linear or branched ureidoalkyl group, a linear or branched guanidinoalkyl group, a linear or branched imidazoloalkyl group or a linear or branched indolylalkyl group, the alkyl portions of these last four groups comprising from one to four carbon atoms.

Preferably, the aromatic group is a C₆ aryl or C₇₋₁₀ aralkyl group, the aromatic nucleus optionally being substituted with one or more C₁₋₄ alkyl or hydroxyl groups.

As amino acids that may be used in the present invention, mention may be made especially of aspartic acid, glutamic acid, alanine, asparagine, glutamine, glycine, histidine, isoleucine, leucine, methionine, N-phenylalanine, proline, serine, threonine, tryptophan, tyrosine, valine, hydroxyproline and mixtures thereof.

Preferably, the amino acids comprise only one amine function, which may or may not be engaged in a ring, and only one acid function. The amino acids that are particularly preferred in the present invention are alanine, asparagine, glutamine, glycine, isoleucine, leucine, methionine, N-phenylalanine, proline, serine, threonine, tyrosine and valine.

The compositions used in the process of the invention have an amino acid concentration of between 0.005% and 20% by weight and particularly between 0.1% and 10% by weight relative to the total weight of the composition.

The composition used in the process of the present invention contains neither a reducing agent nor a source of carbonate ions of the formula: 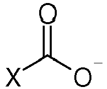 wherein
X is a group selected from the group consisting of O⁻, OH, NH₂, O-OH, and O-COO⁻.

The pH of the composition may range from 6 to 11, preferably between 6.0 and 9.0, and more preferably between 6.0 to 8.0. Since the pH of the composition is not relatively high or low, damage to the keratin fibers by the composition can be reduced.

In order to adjust the pH, an acidic or alkali agent(s) other than sources of ions of the invention may be used alone or in combination. The amount of the acidic or alkali agent(s) is not limited, but may be from 0.1 to 5% by weight relative to the total weight of the composition. As the acidic agents, mention may be made of any inorganic or organic acids which are commonly used in cosmetic products such as citric acid, lactic acid, phosphoric acid or hydrochloric acid (HCl). HCl is preferable. As the alkali agents, mention may be made of any inorganic or organic basic agents which are commonly used in cosmetic products such as ammonia; alcanolamines such as mono-, di- and triethanolamine, isopropanolamine; sodium and potassium hydroxides; urea, guanidine and their derivatives; basic amino acids such as lysine or arginine; and diamines such as those described in the structure below: 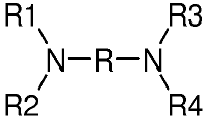 wherein R denotes an alkylene such as propylene optionally substituted by a hydroxyl or a C₁-C₄ alkyl radical, and R₁, R₂, R₃ and R₄ independently denote a hydrogen atom, an alkyl radical or a C₁-C₄ hydroxyalkyl radical, which may be exemplified by 1,3-propanediamine and derivatives thereof. Arginine and monoethanolamine are preferred.

The composition used in the process of the present invention may also comprise one or more additional cosmetic agent(s). The amount of the additional cosmetic agent(s) is not limited, but may be from 0.1 to 10% by weight relative to the total weight of the composition. The cosmetic agent(s) may be selected from the group consisting of volatile or non volatile, linear or cyclic, amine-type or not, silicones, cationic, anionic, non ionic or amphoteric polymers, peptides and derivatives thereof, protein hydrolyzates other than the amino acids of the present invention, synthetic or natural waxes, and especially fatty alcohols, swelling agents and penetrating agents, as well as other active compounds, such as anionic, cationic, non ionic, amphoteric or zwitterionic surfactants, agents for combating hair loss, anti-dandruff agents, associative-type or not, natural or synthetic thickeners, suspending agents, sequestering agents, opacifying agents, dyes, sunscreen agents, fillers, vitamins or provitamins, mineral, vegetable or synthetic oils, as well as fragrances, preserving agents, stabilizers, and mixtures thereof.

The vehicle for the composition used in the process of the present invention is preferably an aqueous medium consisting of water and may advantageously contain one or several cosmetically acceptable organic solvents, which particularly include alcohols, such as ethyl alcohol, isopropyl alcohol, benzyl alcohol and phenylethyl alcohol, or polyols or polyol ethers, such as ethylene glycol monomethyl, monoethyl and monobutyl ethers, propylene glycol or ethers thereof, such as propylene glycol monomethylether, butylene glycol, dipropylene glycol as well as diethylene glycol alkyl ethers, such as diethylene glycol monoethylether or monobutylether. The water may be present in a concentration of from 10 to 90% by weight relative to the total weight of the composition. The organic solvent(s) may then be present in a concentration of from 0.1 to 20% by weight, and preferably from 1 to 10% by weight relative to the total weight of the composition.

The composition used in the process of the present invention may exist in any form such as a lotion, a gel, thickened or not, or a cream. (Keratin Fiber Treatment Process)

The process for treating keratin fibers can be performed by applying onto the keratin fibers a composition comprising at least one amino acid except for cystein, basic arginine and basic lysine, as described above;
then placing the keratin fibers in an occlusive space; and
then heating the keratin fibers,
According to the present description relating to the treatment process for keratin fibers, keratin fibers such as hair are subjected to a specific heating process which is performed in an occlusive space.

The heating process can be performed by any heating means which can be freely controlled to realize the temperature desired for the process.

The heating process may preferably be performed by using a special heating device or devices that can form an occlusive space to restrict the evaporation of evaporable components such as water in the above-described composition from keratin fibers and keep a predetermined temperature in the heating device throughout the process.

If the evaporable components such as water in the above-described composition evaporate from the keratin fibers, most of the heat energy applied to the keratin fibers will be consumed by the evaporation, and therefore the temperature of the keratin fibers cannot increase up to the predetermined temperature until all evaporable components in the composition evaporate.

The above heating device may comprise a heat energy source being either in contact with keratin fibers or apart from keratin fibers, and at least one means to form an occlusive space surrounding the keratin fibers.

The heat energy source is used to heat keratin fibers. The heat energy source may be at least one heater providing at least one selected from the group consisting of hot air, hot steam, high frequency induction heating, microwave heating, infrared ray irradiation, laser, and flash lamp irradiation.

The occlusive space is formed by at least one coating means. A plurality of coating means may be used. The coating means is flexible.

The coating means comprises at least one member selected from the group consisting of a film and a sheet. The material of the film or the sheet is not limited. For example, the film or the sheet may comprise a thermoplastic or thermosetting resin, a paper, a textile, a bonnet, a metal foil such as aluminum foil.

For example, the film or sheet may be set on a heating rod, a heating bar or a heating plate which is covered by keratin fibers.

The coating means may comprise the heat energy source. Therefore, for example, the film or sheet which includes a heater may be set on a rod, a bar, or a plate which is covered by keratin fibers.

The occlusive conditions can restrict the evaporation of evaporable components such as water in the above-described composition applied to keratin fibers, and therefore the temperature of the keratin fibers can be increased higher than that obtainable by a conventional heating process or device for the keratin fibers in open conditions. Furthermore, the keratin fibers can be heated effectively, and the keratin fibers can be heated evenly.

According to one variant, the occlusive space may comprise apertures, the surface area of which is less than 5%, preferably less than 3% and more particularly less than 0.5% of the total surface area of the coating means. According to this variant, the total surface area of the coating means comprises the surface area of, when it is present, an opening means for the coating means.

The apertures may be passages, holes or orifices, which may allow an exchange of air between the occlusive space and the exterior thereof, especially when the reaction such as forming vapor inside the occlusive space is too great. On the other hand, a person skilled in the art could form the apertures such that the diffusion of heat in the occlusive space is not impaired.

The keratin fibers are heated at 60°C to 250ºC, preferably 60ºC to 200ºC, more preferably 60ºC to 150ºC, more preferably 60ºC to 90ºC, during the step of heating the keratin fibers.

The heating process may be performed for an appropriate time which is required to treat keratin fibers. The time length for the heating process is not limited, but it may be from 1 minute to 2 hours, preferably 1 minute to 1 hour, and more preferably 1 minute to 30 minutes. For example, the time for heating may be from 5 to 20 minutes, preferably 10 to 15 minutes.

The keratin fibers may be rinsed after the step of applying the composition onto the keratin fibers and/or after the step of heating the keratin fibers.

### (Permanent Deformation Process for Keratin Fiber)

According to the present invention relating to the treatment process for keratin fibers, the keratin fiber is subjected to mechanical tension which is typically used for permanent deformation.

The permanent deformation process for keratin fibers when mechanical tension is applied to keratin fibers may be performed as follows.

First, keratin fibers are subjected to mechanical tension for deformation. The mechanical tension can be applied to the keratin fibers by any means to deform the keratin fibers to an intended shape. For example, the mechanical tension may be provided by at least one reshaping means selected from the group consisting of a curler, a roller, a clip, a plate and an iron. The reshaping means may comprise at least one heater as described above. If the keratin fibers are rolled around a curler, this rolling-up may be performed on the entire length of the keratin fibers or, for example, on half the length of the keratin fibers. Depending on, for example, the desired hairstyle shape and amount of curls, the rolling-up may be performed with more or less thick locks.

Next, the above-described composition is applied to the keratin fibers. The application of the composition may be performed by any means, such as a brush and a comb. The keratin fibers to which the mechanical tension has been applied should be treated with the composition. It may be possible that the keratin fibers are left as they are for a certain amount of time, if necessary.

Lastly, the above-described heating process is performed. The heat energy is applied to the keratin fibers under occlusive conditions as described above.

This process for permanent deformation of keratin fibers may be performed without any step of oxidizing the keratin fibers. Therefore, the time required for the process according to the present invention can be shorter than that for a conventional process which needs an oxidizing step. Furthermore, damage to the keratin fibers by the oxidizing step can be avoided.

The keratin fibers may be rinsed after the step of applying the composition onto the keratin fibers and/or after the step of heating the keratin fibers.

One embodiment of the hair treatment process according to the present invention may be a process for permanent deforming keratin fibers, in particular hair, comprising:
a) a step of placing the keratin fibers under mechanical tension by rolling them up on at least one reshaping or mechanically tensioning means so as to form curls;
b) a step of applying the above-described composition to the keratin fibers;
c) an optional step of rinsing the keratin fibers,
d) a step of placing at least one coating means on the reshaping or mechanically tensioning means or vice versa to form one or more occlusive spaces; and
e) a step of heating the keratin fibers at a temperature of between 60 ± 2 or 3°C and 250 ± 2 or 3°C for 1 minute to 2 hours.

In this process, the temperature can be set, adjusted and regulated by using one or more heating means, and may be measured with a thermo-measurement probe such as Digital Surface Sensor Module, reference MT-144, sold by Sakaguchi E.H VOC Corp (Japan), set on the keratin fibers. Normally, the probe is set on a single keratin fiber. However, it is advantageous that the probe is set on the part of the keratin fibers which directly contacts with the occlusive space, and more preferably, the probe is set on the part of the keratin fibers which directly contacts with the occlusive space and forms the curl end of the keratin fibers, if a curler is used.

Preferably, the temperature is measured at atmospheric pressure of 101325 Pa.

According to the present invention, the temperature of the keratin fibers may be constant with a fluctuation of ± 2 or 3°C over the head, if the keratin fibers are hair, of an individual, and the probe may be set on any type of keratin fibers.

If the keratin fibers are hair, according to the present invention, the constant temperature with a fluctuation of ± 2 or 3°C can be obtained for any type of hair, and the temperature of the hair can be controlled to be constant ± 2 or 3°C during the heating of the hair at a certain temperature. Thus, the hair style becomes uniform and homogeneous for the entirety of the hair, and a more excellent hair style can be finally obtained.

Advantageously, the coating means may comprise one or more thermal insulating materials, and more advantageously, the coating means may consist of the material(s).

The term "thermal insulating material" means any material which has an electric conductivity of 0 to 1 W/m°C (PVC: 0.17 W/m°C).

Preferably, the heating means may be adjusted such that the temperature measured on the keratin fibers is 50°C or more, more preferably 55°C to less than 150°C, and further more preferably less than 100°C. It is preferable that the heating is performed by heating via electrical resistance.

Advantageously, the coating means is impermeable with regard to the composition used in the step b).

In the above embodiment, at least one of the reshaping or mechanical tensioning means and at least one of the covering means may include a heater.

In the above embodiment, "occlusive space" means that when the coating means is placed on the reshaping or mechanical tensioning means, or vice versa, they together form a closed structure in which heat can diffuse, but heat cannot diffuse out of or is difficult to diffuse out of the closed structure. It is preferable that the coating means and the reshaping or mechanical tensioning means can form the occlusive space when they are set on the head, if the keratin fibers are hair.

The occlusive space may form a condensation cage in which water and a component or components in the composition used in the step b) may evaporate from the keratin fibers, adhere to the wall of the coating means, and drop onto the keratin fibers. This cycle may be repeated during the heating of the keratin fibers. Thus, the keratin fibers can be always kept wet, and drying and deteriorating of the keratin fibers will be prevented.

The formation of occlusive space is an important characteristic of the present invention, because the keratin fibers in the occlusive space can be kept wet and the temperature of the keratin fibers can be constant.

Preferably, the process of the present invention may comprise an additional step of tightening the coating means on the head of an individual, if the keratin fibers are hair, by an elastic cord, an extensible band, or a stretch.

According to the process of the present invention, because of the occlusive space in which the composition can be continuously condensed on the keratin fibers, the amount of a cosmetic component or components in the composition is advantageously reduced as compared to the processes in the prior art. The amount of the cosmetic component(s) may be 0.3 to 3wt% of the composition.

In a preferred embodiment, a coating means may be placed on each hair curler as the reshaping or mechanically tensioning means, if the keratin fibers are hair. In other words, each of the hair curlers, if two or more hair curlers are used, may be covered individually by a coating means. It is advantageous to cover each hair curler because leaking to the scalp of the composition which has been applied onto keratin fibers in the step b) can be prevented.

In another preferred embodiment, a coating means may cover all hair curlers, if two or more hair curlers are used. In other words, the coating means may cover the entirety of the head if the keratin fibers are hair.

Advantageously, the occlusive space formed in the step d) may be maintained during the step e). In other words, the coating means may be removed only after the step e) or after the stop of the heating in the step e).

If necessary, the composition may be applied to keratin fibers before applying mechanical tension to the keratin fibers. It may be possible that the keratin fibers are left as they are for a certain amount of time, if necessary, before and/or after applying mechanical tension to the keratin fibers, before and/or after applying the above-described composition to the keratin fibers, and before and/or after heating the keratin fibers.

After the above step e), if necessary, the keratin fibers may be fixed by oxidation after being taken out from the coating means.

### (Products)

It is also described a composition for treating keratin fibers to be heated in an occlusive space, comprising at least one amino acid except for cystein, basic arginine and basic lysine,
wherein
the composition contains neither a reducing agent nor a source of carbonate ions of the formula: 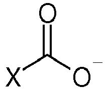 wherein
X is a group selected from the group consisting of O⁻, OH, NH₂, O-OH, and O-COO⁻.

This composition may not need to be used in combination with an oxidizing agent which is used in a conventional permanent deformation of keratin fibers. Therefore, if keratin fibers should be permanently deformed, the composition may be used in one step, whereas two steps (reducing step and oxidizing step) are necessary in the conventional permanent deformation of keratin fibers.

This composition may have the same technical features as those of the composition described above.

It is also described a kit for treating keratin fibers, comprising:
a device comprising
   at least one reshaping means to provide the keratin fibers with mechanical tension,
   at least one coating means to form an occlusive space, and
   at least one heater to heat the keratin fibers in the occlusive space;
and
a composition comprising at least one amino acid except for cystein, basic arginine and basic lysine, wherein the composition contains neither a reducing agent nor a source of carbonate ions of the formula: 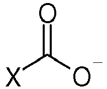
wherein
X is a group selected from the group consisting of O⁻, OH, NH₂, O-OH, and O-COO⁻.

The coating means and the heater, as well as the composition in the kit, may be the same as those described above.

### EXAMPLES

The present invention will be described in more detail by way of examples, which however should not be construed as limiting the scope of the present invention.

### Composition 1

A hair treatment composition (referred to as "Composition 1") having the following composition shown in Table 1 was prepared (active ingredients in wt %).

**Table 1**

| | |
|---|---|
| Proline | 5 |
| Methionine | 2 |
| pH adjuster (NaOH) | qsp pH 8.5 |
| Water | qsp 100 |

### Example 1

Composition 1 was applied to a 1g natural Japanese hair swatch previously wrapped on a 1.7 cm heating perm-roller for 15 minutes. Then, the perm-roller was covered by a plastic film and plugged into a Digital Perm Machine (Oohiro, model ODIS-2). After the heating process at 90°C for 15 minutes, the hair was rinsed, removed from the perm-roller and dried.

The hair was soft, smooth and detangled.

### Comparative Example 1

Composition 1 was applied to a 1g natural Japanese hair swatch which was the same as that used in Example 1 for 15 minutes. After leaving the hair without heating for 15 minutes, the hair was rinsed and dried.

No specific change was observed after the treatment.

### Composition 2

A hair treatment composition (referred to as "Composition 2") having the following composition shown in Table 2 was prepared (active ingredients in wt %) .

**Table 2**

| | |
|---|---|
| Histidine | 3 |
| Glycine | 10 |
| pH adjuster | qsp pH 7.5 |
| Water | qsp 100 |

### Example 2

Composition 2 was applied to a 1g natural Japanese hair swatch previously wrapped on a 1.7 cm heating perm-roller for 15 minutes. Then, the perm-roller was covered by a plastic film and plugged onto a Digital Perm Machine (Oohiro, model ODIS-2). After the heating process at 90°C for 15 minutes, the hair was rinsed, removed from the perm-roller and dried.

Hair is smooth and shiny.

### Comparative Example 2

Composition 2 was applied to a 1g natural Japanese hair swatch which was the same as that used in Example 2 for 15 minutes. After leaving the hair without heating for 15 minutes, the hair was rinsed and dried.

No specific change was observed after the treatment.

### Composition 3

A hair treatment composition (referred to as "Composition 3") having the following composition shown in Table 3 was prepared (active ingredients in wt %) .

**Table 3**

| | |
|---|---|
| Leucine | 1.5 |
| Phenyalanine | 1 |
| Threonine | 10 |
| pH adjuster (NaOH) | qsp pH 8 |
| Water | qsp 100 |

### Example 3

Composition 3 was applied to a natural 1g Japanese hair swatch previously wrapped on a 1.7 cm heating perm-roller for 15 minutes. Then, the perm-roller was covered by a plastic film and plugged onto a Digital Perm Machine (Oohiro, model ODIS-2). After the heating process at 90°C for 15 minutes, the hair was rinsed, removed from the perm-roller and dried.

The hair was smooth, detangled and shiny.

### Comparative Example 3

Composition 3 was applied to a 1g natural Japanese hair swatch which was the same as that used in Example 3 for 15 minutes. After leaving the hair without heating for 15 minutes, the hair was rinsed and dried.

No specific change was observed after the treatment.

## Claims

1. A permanent deformation process for keratin fibers comprising the steps of:
providing the keratin fibers with mechanical tension;
applying onto the keratin fibers a composition comprising at least one amino acid except for cystein, basic arginine and basic lysine, in an amount from 0.005 to 20% by weight relative to the total weight of the composition;
then placing the keratin fibers in an occlusive space surrounding the keratin fibers which is formed by at least one coating means to restrict the evaporation of evaporable components in the composition from the keratin fibers and keep the keratin fibers wet; and
then heating the keratin fibers at a temperature ranging from 60 to 250°C while keeping the keratin fibers in the wet state,
wherein
the composition contains neither a reducing agent nor a source of carbonate ions of the formula: 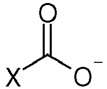 wherein
X is a group selected from the group consisting of O⁻, OH, NH₂, O-OH, and O-COO⁻, wherein the coating means is flexible and comprises at least one member selected from the group consisting of a film and a sheet, and
if the occlusive space comprises apertures, the surface area of said apertures is less than 5% of the total surface area of the coating means.

2. The process according to Claim 1, further comprising the step of rinsing the keratin fibers after the step of applying the composition onto the keratin fibers and/or after the step of heating the keratin fibers.

3. The process according to Claim 1 or 2, wherein the step of providing the keratin fibers with mechanical tension is performed after the step of applying onto the keratin fibers a composition comprising at least one amino acid except for cystein, basic arginine and basic lysine.

4. The process according to any one of Claims 1 to 3, wherein the keratin fibers are heated at 60ºC to 200ºC during the step of heating the keratin fibers.

5. The process according to any one of Claims 1 to 4, wherein the keratin fibers are heated by at least one heater providing at least one selected from the group consisting of hot air, hot steam, high frequency induction heating, microwave heating, infra-red ray irradiation, laser, and flash lamp irradiation.

6. The process according to Claim 5, wherein the coating means comprises the heater.

7. The process according to any one of Claims 1 to 6, wherein the amino acid is an alpha-amino acid.

8. The process according to any one of Claims 1 to 7, wherein the amino acid corresponds to the formula: 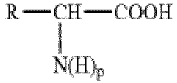 in which
when p=2, R represents a hydrogen atom, an aliphatic group optionally containing one or several nitrogen atoms, a heterocyclic portion, or an aromatic group, or
when p=1, R can form a heterocycle with the nitrogen atom of -N(H)ₚ.

9. The process according to any one of Claims 1 to 8, wherein the amino acid is selected from the group consisting of aspartic acid, glutamic acid, alanine, asparagine, glutamine, glycine, histidine, leucine, isoleucine, methionine, N-phenylalanine, proline, hydroxyproline, serine, threonine, tryptophan, tyrosine, valine, and mixtures thereof.

10. The process according to any one of Claims 1 to 9, wherein the composition comprises the amino acid in an amount of 0.1 to 10% by weight relative to the total weight of the composition.

## Patentansprüche

1. Permanentverformungsverfahren für Keratinfasern, das die Schritte umfasst:
Anlegen einer mechanischen Spannung an die Keratinfasern;
Anwenden einer Zusammensetzung auf die Keratinfasern, die zumindest eine Aminosäure außer Cystein, basischem Arginin und basischem Lysin umfasst, in einer Menge von 0,005 bis 20 Gew.-% bezogen auf das Gesamtgewicht der Zusammensetzung;
danach Platzieren der Keratinfasern in einem okklusiven Raum, der die Keratinfasern umgibt, der durch zumindest ein Beschichtungsmittel gebildet wird, um die Verdampfung verdampfbarer Komponenten in der Zusammensetzung aus den Keratinfasern zu beschränken und die Keratinfasern nass zu halten; und
danach Erhitzen der Keratinfasern bei einer Temperatur im Bereich von 60 bis 250 °C, während die Keratinfasern im nassen Zustand gehalten werden,
wobei
die Zusammensetzung weder ein Reduktionsmittel noch eine Quelle von Carbonationen der Formel: 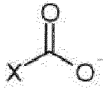 enthält,
wobei
X eine Gruppe ist, die aus der Gruppe ausgewählt ist, bestehend aus O⁻, OH, NH₂, O-OH und O-COO⁻,
wobei das Beschichtungsmittel flexibel ist und zumindest ein Element umfasst, das aus der Gruppe ausgewählt ist, bestehend aus einem Film und einer Folie, und
wenn der okklusive Raum Öffnungen umfasst, der Oberflächenbereich der Öffnungen weniger als 5 % des gesamten Oberflächenbereichs des Beschichtungsmittels ist.

2. Verfahren nach Anspruch 1, das ferner den Schritt des Spülens der Keratinfasern nach dem Schritt des Anwendens der Zusammensetzung auf die Keratinfasern und/oder nach dem Schritt des Erhitzens der Keratinfasern umfasst.

3. Verfahren nach Anspruch 1 oder 2, wobei der Schritt des Anlegens einer mechanischen Spannung an die Keratinfasern nach dem Schritt des Anwendens einer Zusammensetzung auf die Keratinfasern, die zumindest eine Aminosäure außer Cystein, basischem Arginin und basischem Lysin umfasst, durchgeführt wird.

4. Verfahren nach einem der Ansprüche 1 bis 3, wobei die Keratinfasern bei 60 °C bis 200 °C während des Schritts des Erhitzens der Keratinfasern erhitzt werden.

5. Verfahren nach einem der Ansprüche 1 bis 4, wobei die Keratinfasern durch zumindest ein Heizmittel erhitzt werden, das zumindest eines bereitstellt, das aus der Gruppe ausgewählt ist, bestehend aus heißer Luft, heißem Dampf, Hochfrequenzinduktionserhitzung, Mikrowellenerhitzung, Infrarotstrahlenbestrahlung, Laser und Blitzlampenbestrahlung.

6. Verfahren nach Anspruch 5, wobei das Beschichtungsmittel das Heizmittel umfasst.

7. Verfahren nach einem der Ansprüche 1 bis 6, wobei die Aminosäure eine Alpha-Aminosäure ist.

8. Verfahren nach einem der Ansprüche 1 bis 7, wobei die Aminosäure der Formel entspricht: 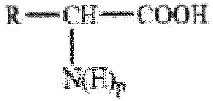 wobei
wenn p 2 ist, R ein Wasserstoffatom, eine aliphatische Gruppe, die optional ein oder mehrere Stickstoffatome enthält, einen heterocyclischen Abschnitt oder eine aromatische Gruppe darstellt, oder
wenn p 1 ist, R einen Heterocyclus mit dem Stickstoffatom von -N(H)ₚ- bilden kann.

9. Verfahren nach einem der Ansprüche 1 bis 8, wobei die Aminosäure aus der Gruppe ausgewählt ist, bestehend aus Asparaginsäure, Glutaminsäure, Alanin, Asparagin, Glutamin, Glycin, Histidin, Leucin, Isoleucin, Methionin, N-Phenylalanin, Prolin, Hydroxyprolin, Serin, Threonin, Tryptophan, Tyrosin, Valin und Mischungen davon.

10. Verfahren nach einem der Ansprüche 1 bis 9, wobei die Zusammensetzung die Aminosäure in einer Menge von 0,1 bis 10 Gew.-% bezogen auf das Gesamtgewicht der Zusammensetzung umfasst.

## Revendications

1. Procédé de déformation permanente de fibres de kératine, comprenant les étapes suivantes :
soumission des fibres de kératine à une tension mécanique ;
application sur les fibres de kératine d'une composition comprenant au moins un acide aminé à l'exception de la cystéine, de l'arginine basique et de la lysine basique, en une quantité de 0,005 à 20 % en poids par rapport au poids total de la composition ;
puis placement des fibres de kératine dans un espace occlusif entourant les fibres de kératine, qui est formé par au moins un moyen de revêtement pour limiter l'évaporation des composants évaporables dans la composition hors des fibres de kératine et maintenir les fibres de kératine humides ; et
ensuite chauffage des fibres de kératine à une température située dans la plage allant de 60 à 250°C tout en maintenant les fibres de kératine à l'état humide,
dans lequel
la composition ne contient ni agent réducteur ni source d'ions carbonate de formule : 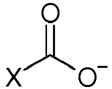 dans laquelle X est un groupe choisi dans l'ensemble constitué par O⁻, OH, NH₂, O-OH, et O-COO⁻ ;
dans lequel le moyen de revêtement est flexible et comprend au moins un membre choisi dans l'ensemble constitué par un film et une feuille, et
si l'espace occlusif comprend des ouvertures, la superficie desdites ouvertures est inférieure à 5 % de la superficie totale du moyen de revêtement.

2. Procédé selon la revendication 1, comprenant en outre l'étape de rinçage des fibres de kératine après l'étape d'application de la composition sur les fibres de kératine et/ou après l'étape de chauffage des fibres de kératine.

3. Procédé selon la revendication 1 ou 2, **caractérisé en ce que** l'étape de soumission des fibres de kératine à une tension mécanique est effectuée après l'étape d'application sur les fibres de kératine d'une composition comprenant au moins un acide aminé à l'exception de la cystéine, de l'arginine basique et de la lysine basique.

4. Procédé selon l'une quelconque des revendications 1 à 3, dans lequel les fibres de kératine sont chauffées à une température de 60°C à 200°C durant l'étape de chauffage des fibres de kératine.

5. Procédé selon l'une quelconque des revendications 1 à 4, dans lequel les fibres de kératine sont chauffées par au moins un dispositif chauffant délivrant au moins l'un choisi dans l'ensemble constitué par de l'air chaud, de la vapeur chaude, un chauffage par induction haute fréquence, un chauffage par micro-ondes, une irradiation de rayons infrarouges, un laser, et une irradiation par lampe flash.

6. Procédé selon la revendication 5, dans lequel le moyen de revêtement comprend le dispositif chauffant.

7. Procédé selon l'une quelconque des revendications 1 à 6, dans lequel l'acide aminé est un acide alpha-aminé.

8. Procédé selon l'une quelconque des revendications 1 à 7, dans lequel l'acide aminé correspond à la formule : 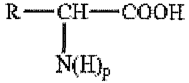 dans laquelle
quand p = 2, R représente un atome d'hydrogène, un groupe aliphatique contenant éventuellement un ou plusieurs atomes d'azote, un fragment hétérocyclique, ou un groupe aromatique, ou
quand p = 1, R peut former un hétérocycle avec l'atome d'azote de -N(H)ₚ.

9. Procédé selon l'une quelconque des revendications 1 à 8, dans lequel l'acide aminé est choisi dans l'ensemble constitué par l'acide aspartique, l'acide glutamique, l'alanine, l'asparagine, la glutamine, la glycine, l'histidine, la leucine, l'isoleucine, la méthionine, la N-phénylalanine, la proline, l'hydroxyproline, la sérine, la thréonine, le tryptophane, la tyrosine, la valine, et leurs mélanges.

10. Procédé selon l'une quelconque des revendications 1 à 9, dans lequel la composition comprend l'acide aminé en une quantité de 0,1 à 10 % en poids par rapport au poids total de la composition.
